Europäisches Patentamt

European Patent Office

Office européen des brevets

(11) Veröffentlichungsnummer: **0 360 322 B1**

(12) **EUROPÄISCHE PATENTSCHRIFT**

(45) Veröffentlichungstag der Patentschrift: **01.12.93**

(51) Int. Cl.⁵: **A61B 6/12**

(21) Anmeldenummer: **89202238.5**

(22) Anmeldetag: **05.09.89**

(54) **Verfahren zum Positionieren eines auf einer Tischplatte eines Lagerungstisches befindlichen Patienten und Gerät zum Durchführen des Verfahrens.**

(30) Priorität: **06.09.88 DE 3830183**

(43) Veröffentlichungstag der Anmeldung:
**28.03.90 Patentblatt 90/13**

(45) Bekanntmachung des Hinweises auf die Patenterteilung:
**01.12.93 Patentblatt 93/48**

(84) Benannte Vertragsstaaten:
**DE FR GB NL**

(56) Entgegenhaltungen:
**EP-A- 0 160 583**
**EP-A- 0 257 429**
**EP-A- 0 269 801**
**US-A- 4 764 944**

**PHYSICS IN MEDICINE & BIOLOGY, Band 25, Nr. 2. März 1980, Seiten 349-355, The Institute of Physics, London, GB; R.A. SHERLOCK et al.: "A method of precision position determination using x-ray stereography"**

(73) Patentinhaber: **Philips Patentverwaltung GmbH**
**Wendenstrasse 35c**
**D-20097 Hamburg(DE)**

(84) Benannte Vertragsstaaten:
**DE**

(73) Patentinhaber: **N.V. Philips' Gloeilampenfabrieken**
**Groenewoudseweg 1**
**NL-5621 BA Eindhoven(NL)**

(84) Benannte Vertragsstaaten:
**FR GB NL**

(72) Erfinder: **Schwieker, Horst**
**Trenkner Weg 21**
**D-2000 Hamburg 52(DE)**

(74) Vertreter: **Hartmann, Heinrich, Dipl.-Ing. et al**
**Philips Patentverwaltung GmbH,**
**Wendenstrasse 35c**
**D-20097 Hamburg (DE)**

**Beschreibung**

Die Erfindung bezieht sich auf ein Verfahren zum Positionieren eines auf der Tischplatte eines Lagerungstisches befindlichen Patienten, wobei eine erste Röntgenaufnahme mit einer ersten Richtung des Zentralstrahls angefertigt und elektronisch gespeichert wird, wonach die Tischplatte in einem ersten Positioniervorgang in einer ersten und in einer dazu senkrechten zweiten Richtung verfahren wird und die Röntgenaufnahme und eine darin eingeblendete elektronische Marke auf einem Sichtgerät relativ zueinander entsprechend dem Fahrweg der Tischplatte verschoben werden, sowie auf ein Gerät zum Durchführen des Verfahrens.

Bei vielen Untersuchungen und Behandlungen im medizinischen Bereich ist es erforderlich, eine bestimmte Region eines Patienten in eine genau definierte Position zu bringen. So muß beispielsweise bei der Nierensteinzertrümmerung der Patient so im Raum bewegt werden, daß das Konkrement, d.h. der Nierenstein, exakt im Fokus eines Stoßwellenerzeugers zur Zertrümmerung von Konkrementen positioniert wird.

Es ist bekannt, daß bei derartigen Geräten die Ortung und die Positionierung mittels einer Röntgendurchleuchtungseinrichtung erfolgen kann. Dabei wird die Tischplatte unter Durchleuchtungskontrolle aus verschiedenen Perspektiven solange verschoben, bis sich die Abbildung des Nierensteins an einer bestimmten Stelle des Durchleuchtungsbildes befindet, beispielsweise in der Bildmitte, wobei die Stelle zweckmäßigerweise durch eine geeignete elektronische Marke, z.B. ein Fadenkreuz, sichtbar gemacht wird. Nachteilig dabei ist, daß ein solcher Positioniervorgang für den Patienten, aber auch für den Benutzer mit einer relativ großen Strahlenbelastung verbunden ist, weil die Durchleuchtung solange eingeschaltet bleibt, bis sich der Nierenstein in der gewünschten Position befindet.

Bei dem eingangs erwähnten Verfahren bzw. Gerät, das aus der EP-OS 160 583 bekannt ist, wird die Strahlenbelastung verringert, weil die Positionierung mit Hilfe einer einzigen Röntgenaufnahme erfolgt, bei der die Röntgenstrahlung nur kurzzeitig eingeschaltet werden muß. Allerdings kann damit nur dann eine genaue Positionierung erfolgen, wenn das Konkrement sich zufällig schon in der zur Tischplatte parallelen Ebene befindet, in der seine Soll-Position liegt. Anderenfalls ist eine genaue Positionierung nicht möglich, und zwar auch nicht in Längs- und Querrichtung der Tischplatte, weil eine Röntgenaufnahme bekanntlich eine Zentralprojektion darstellt.

Aufgabe der vorliegenden Erfindung ist es, einen Patienten mit geringer Strahlenbelastung genau zu positionieren.

Ausgehend von einem Verfahren der eingangs genannten Art wird diese Aufgabe erfindungsgemäß durch die im Anspruch 1 angegebenen Maßnahmen gelöst. Ein Gerät zur Durchführung dieses Verfahrens ist in Anspruch 4 angegeben.

Bei der Erfindung werden also zwei Röntgenaufnahmen aus unterschiedlicher Perspektive angefertigt, und es erfolgen zwei Positioniervorgänge. Nach dem ersten Positioniervorgang befindet sich der in eine definierte Position zu bringende Bereich des Patienten, z.B. ein Nierenstein, auf einer zur Projektionsrichtung des Nierensteins parallelen Geraden durch diese Position. Wenn die Fahrwege in der ersten und zweiten Richtung so mit dem Fahrweg in der dritten Richtung gekoppelt sind, daß die Fahrwege in den beiden ersten Richtungen dem Produkt aus dem Fahrweg in der dritten Richtung und den Fahrwegen in der ersten und zweiten Richtung am Ende des ersten Positioniervorganges proportional ist, kann erreicht werden, daß der Nierenstein auf dieser Geraden verfahren wird. Da gleichzeitig eine Berechnung der Relativverschiebung der Marke und der Tischplattenverschiebung erfolgt, ist sichergestellt, daß in dem Augenblick, in dem der Nierenstein die Sollposition erreicht hat, die elektronische Marke in der Röntgenaufnahme mit der Abbildung des Nierensteins zusammenfällt, so daß der Benutzer weiß, daß er jetzt den Positioniervorgang beenden kann.

Ein Vorteil der Erfindung ist, daß nach dem ersten Positioniervorgang der Patient schon relativ dicht an der Sollposition ist. Infolgedessen haben Nichtlinearitäten, wie sie u.a. durch die Krümmung des Eingangsschirms des Bildverstärkers, mit dessen Hilfe die Röntgenaufnahmen angefertigt werden können, allenfalls einen geringen Einfluß auf die Genauigkeit der Positionierung. Ein weiterer Vorteil ist, daß die Verschiebung der Tischplatte und der elektronischen Marke in der Röntgenaufnahme durch die gleiche Bedieneinheit gesteuert werden kann, die zur Steuerung der Tischplattenbewegung ohnehin erforderlich ist.

Die unterschiedlichen Perspektiven bei den Röntgenaufnahmen werden nach einer bevorzugten Weiterbildung der Erfindung dadurch erreicht, daß die erste und die zweite Aufnahme mit in unterschiedlichen Richtungen verlaufendem Zentralstrahl angefertigt werden. Dabei muß das Röntgenaufnahmesystem schwenkbar gestaltet sein.

Diese Voraussetzung kann entfallen, wenn nach einer anderen Weiterbildung der Erfindung vorgesehen ist, daß die beiden Röntgenaufnahmen mit der gleichen Richtung des Zentralstrahls angefertigt werden und daß nach dem ersten Positioniervorgang und vor der zweiten Röntgenaufnahme die Tischplatte um eine definierte Strecke in der ersten bzw. der zweiten Richtung verschoben wird. Der Zentralstrahl hat dabei zwar bei beiden Aufnahmen die gleiche Richtung, jedoch erscheint das Konkrement infolge der Verschiebung nach dem ersten Positioniervorgang in unterschiedlicher Perspektive. Allerdings ist dieses Verfahren nicht ganz so genau wie das zuvor erläuterte Verfahren.

Die Erfindung soll nachstehend anhand der Zeichnungen näher erläutert werden. Es zeigen:

Fig. 1 ein Röntgengerät, mit dem die Erfindung ausführbar ist,

Fig. 2 ein Prinzipschaltbild der Tischplattensteuerung bei einem solchen Gerät und Fig. 3 die geometrischen Verhältnisse vor und nach den Positioniervorgängen.

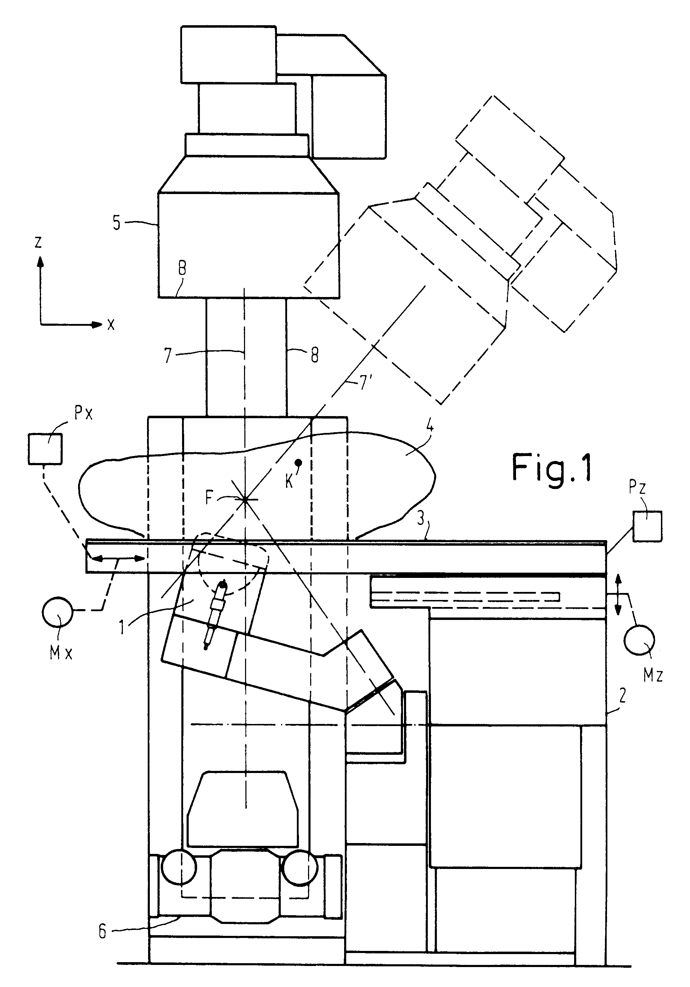

In Fig. 1 ist ein Gerät für die Nierenlithotripsie dargestellt, das die Ortung von Konkrementen (Nierensteinen) ihre Positionierung im Fokus eines Stoßwellenerzeugers und ihre Zertrümmerung erlaubt. Das Gerät weist einen Stoßwellenerzeuger 1 auf, der in einem Fokus F eine Stoßwelle erzeugen kann. Der zu untersuchende Patient 4 befindet sich auf einem Lagerungstisch 2, der mit einer Tischplatte 3 versehen ist, die in x-Richtung (d.h. horizontal in der Zeichenebene von Fig. 1), in y-Richtung (senkrecht zur Zeichenebene) und in z-Richtung (vertikal) verschoben werden kann. Die Tischplatte muß so verschoben werden, daß das Konkrement K im Körper des Patienten 4 in den Fokus F des Stoßwellenerzeugers 1 gelangt.

Zur Verschiebung der Tischplatte in x-Richtung ist ein Antriebsmotor Mx vorgesehen, der zusammen mit einem die Position der in x-Richtung messenden Positionsgeber Px einen Teil eines Regelkreises bildet. Ebenso gibt es einen Antriebsmotor Mz zur Verschiebung der Tischplatte 3 in z-Richtung und einen zugehörigen Positionsgeber Pz. Der Antriebsmotor My und der Positionsgeber Py für die y-Richtung ist in Fig. 1 nicht dargestellt.

Die Ortung und die Positionierung erfolgt mit Hilfe einer Röntgenaufnahmeanordnung, die einen Bildwandler 5 in Form eines Bildverstärkers und eine Strahlenquelle in Form eines Röntgenstrahlers 6 aufweist. Das von dem Röntgenstrahler 6 emittierte Strahlenbündel ist auf die Eingangfläche B des Bildverstärkers 5 ausgerichtet. Der Zentralstrahl 7, der die Mitte des Bildverstärkers mit dem Brennfleck der Strahlenquelle verbindet, verläuft in der Ausgangsstellung vertikal. Bei der Ortung bzw. Positionierung wird der Stoßwellenerzeuger aus dem Strahlengang entfernt.

Der Röntgenstrahler 6 und der Bildverstärker 5 sind über einen Tragarm 8 miteinander verbunden, der um eine in y-Richtung durch den Fokus F verlaufende Achse schwenkbar ist. In Fig. 1 ist der Bildverstärker 5 zusätzlich gestrichelt in einer geschwenkten Position dargestellt, wobei der Zentralstrahl 7' den vertikalen Zentralstrahl 7 im Fokus F schneidet.

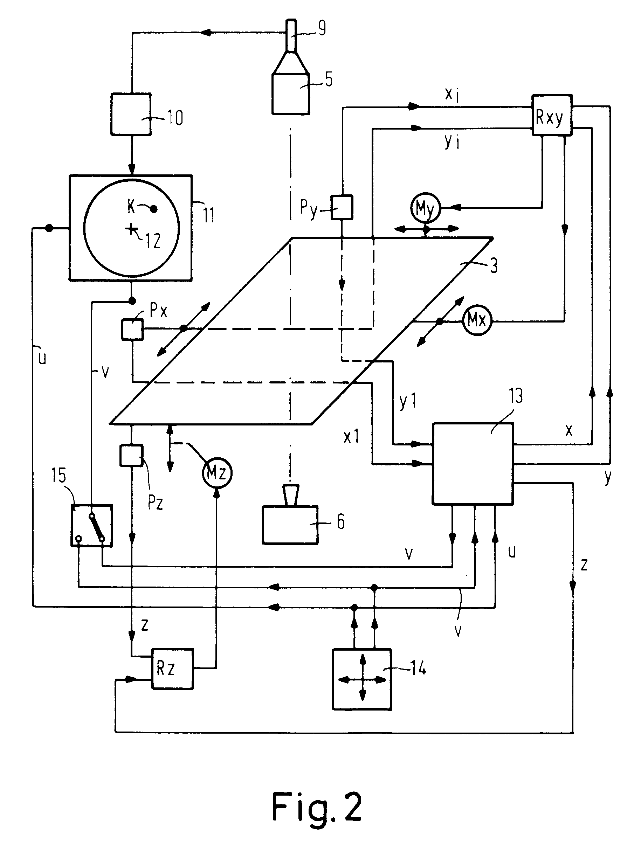

Wie sich aus Fig. 2 ergibt, ist an den Ausgang des Bildverstärkers 5 eine Fernsehkamera 9 angeschlossen, deren Videosignal nach einer Röntgenaufnahme in einem Speicher 10 gespeichert wird. Die gespeicherte Röntgenaufnahme wird auf einem Sichtgerät bzw. Monitor 11 wiedergegeben. In die Röntgenaufnahme wird zur Anzeige der Position des Fokus 12 elektronisch eine Marke eingeblendet, beispielsweise ein Fadenkreuz 12. Bei einer Verschiebung der Tischplatte werden die Röntgenaufnahme und das Fadenkreuz 12 auf dem Monitor relativ zueinander verschoben. Diese Relativverschiebung kann dadurch erfolgen, daß die Röntgenaufnahme verschoben wird, während das Fadenkreuz 12 stehen bleibt. Günstiger ist es aber, die Röntgenaufnahme auf dem Monitor nicht zu bewegen und lediglich das Fadenkreuz zu verschieben. Das Fadenkreuz 12 bewegt sich also auf das Kronkrement K in der Röntgenaufnahme zu, während das Konkrement K im Patienten sich auf den Fokus F zubewegt. Zur Verschiebung des Fadenkreuzes 12 innerhalb der Röntgenaufnahme werden dem Monitor zwei Signale u und v zugeführt.

Wie bereits erwähnt, bilden die Antriebsmotoren Mx, My und Mz für die Verschiebung der Tischplatte in x- y- und z-Richtung zusammen mit den zugehörigen Positionsgebern Px, Py und Pz sowie mit Reglern Rxy und Rz Regelkreise, die die Position der Tischplatte bestimmen. Die Führungsgrößen x, y und z für diese Regelkreise werden von einer Steuereinheit 13 erzeugt, die aus den Signalen u und v die Signale x, y und z berechnet und die außerdem die am Ende des ersten Positioniervorganges sich in x- und y-Richtung ergebenden Fahrwege x1 und y1 speichert. Die Signale u und v können von einem Koordinatenschalter 14 geliefert werden.

Die Verschiebung der Tischplatte derart, daß das Konkrement K im Fokus F des Stoßwellenerzeugers 1 positioniert wird, erfolgt auf folgende Weise:

Zunächst wird eine erste Röntgenaufnahme angefertigt, wobei die Röntgenaufnahmeanordnung 5, 6 die in Fig. 1 in ausgezogenen Linien dargestellte Position einnimmt, bei der der Zentralstrahl 7 vertikal und senkrecht zur Tischplatte 3 verläuft. Die Röntgenaufnahme wird im Speicher 10 gespeichert und auf dem Monitor 11 wiedergegeben. Die Tischplatte wird in x- und y-Richtung verfahren, und das Fadenkreuz 12 wird proportional zur Bewegung des Patienten auf dem Bildschirm 11 verschoben.

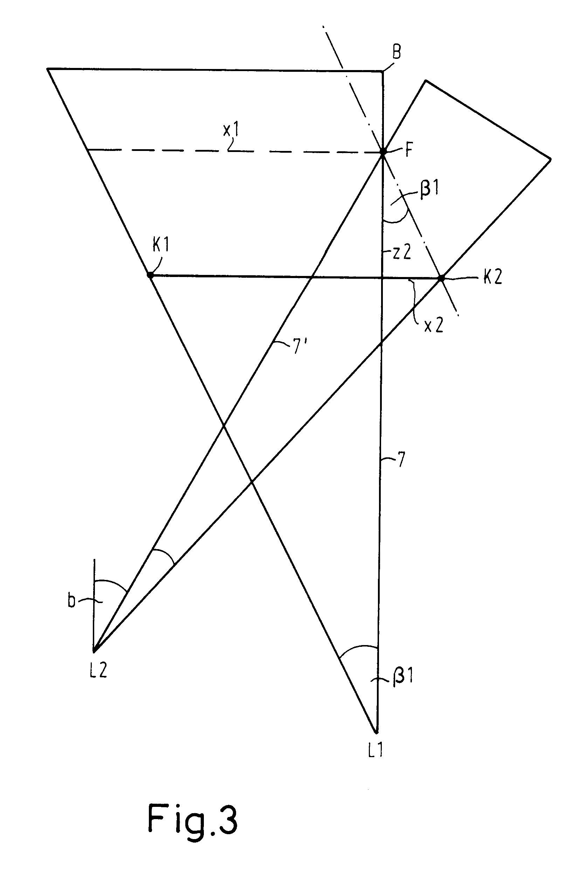

In Fig. 3 sind die geometrischen Verhältnisse bei den Röntgenaufnahmen dargestellt, wobei allerdings zur Verdeutlichung der Öffnungswinkel des Röntgenstrahlenbündels wesentlich größer ist, als er sich bei der Anordnung nach Fig. 1 ergeben kann. Die Position des Röntgenstrahlers bei der ersten Röntgenaufnahme ist dabei mit L1 bezeichnet, K1 bezeichnet die Lage des Konkrements K vor dem ersten Positioniervorgang und B bezeichnet den Eingangsschirm des Bildverstärkers; allerdings ist dieser nur in dem Bereich zwischen seinen Mittelpunkt und der Projektion des Konkrements K1 durch eine Gerade angedeutet. Der Abstand des Fokus F von der Projektion des Konkrements K1 auf eine zum Zentralstrahl 7 senkrechte und durch den Fokus F verlaufende Ebene ist in Fig. 3 mit x1 bezeichnet und der Winkel, den der das Konkrement K1 treffende Röntgenstrahl mit dem Zentralstrahl 7 einschließt, mit $\beta 1$.

Bei dem auf die erste Röntgenaufnahme folgenden ersten Positioniervorgang wird angenommen, daß das Konkrement sich in einer den Fokus F enthaltenden, zum Zentralstrahl 7 senkrechten Ebene befindet, und die Positionierung erfolgt in der Weise, daß - wenn das Konkrement sich tatsächlich in der zum Zentralstrahl senkrechten und den Fokus F enthaltenden Ebene befände - das Konkrement am Ende des Positioniervorganges im Fokus F positioniert wäre. Der Patient wird also dabei in x-Richtung um die Strecke x1 - und entsprechend in y-Richtung um eine in Fig. 3 nicht dargestellte Strecke y1 - verschoben. Da das Konkrement K1 sich aber in einer Ebene befindet, die von der erwähnten Ebene den Abstand z2 hat, ist die Verschiebung in x- und y-Richtung zu groß, so daß das Konkrement sich am Ende des ersten Positioniervorganges nicht einmal auf dem Zentralstrahl 7 befindet. Die Lage des Konkrements am Ende des ersten Positioniervorganges und vor der zweiten Röntgenaufnahme ist in Fig. 3 mit K2 bezeichnet. Der Abstand zwischen K1 und K2 beträgt x1.

Um kontrollieren zu können, daß das Konkrement am Ende der ersten Aufnahme die erwünschte Position hat, wird gleichzeitig und proportional zu der Tischplattenverschiebung das Fadenkreuz 12 in der Röntgenaufnahme auf dem Monitor 11 durch die Signale u und v verschoben. Zwischen x und y einerseits und u und v anderseits besteht dabei die Beziehung

$$x = a \cdot c \cdot u/d \quad (1)$$

$$y = a \cdot c \cdot v/d \quad (2)$$

Dabei ist a der Abstand zwischen F und L1, d der Abstand der Strahlenquelle L1 von der Eingangsebene B des Bildverstärkers und c ein Proportionalitätsfaktor, der den Quotienten zwischen der Entfernung eines Punktes auf der Eingangsebene des Bildverstärkers 5 von der Bildverstärkermitte einerseits und der Größe des Signals u bzw. v darstellt, das erforderlich ist, um das Fadenkreuz 12 auf dem Monitor mit der Abbildung dieses Punktes in dem Monitor zur Deckung zu bringen.

Somit erzeugt die Steuereinheit 13 aus den vom Koordinatengeber 14 vorgegebenen Signalen u und v die Signale x und y entsprechend den Gleichungen (1) und (2). Wenn das Konkrement die in Fig. 3 mit K2 bezeichnete Lage erreicht hat, fällt in diesem Fall auf dem Monitor das Konkrement K und das Fadenkreuz 12 zusammen - obwohl, wie Fig. 3 zeigt, das Konkrement K2 nicht auf dem Zentralstrahl 7 liegt. Die von den Positionsgebern Px und Py am Ende des Positioniervorganges gelieferten Werte x1 und y1 werden in der Steuereinheit 13 gespeichert.

Wie man aus Fig. 3 erkennt, hat am Ende des ersten Positioniervorganges das Konkrement K2 in x-Richtung den Abstand x2 und in z-Richtung den Abstand z2 vom Fokus F. Da die Verbindungslinie von K2 und F die gleiche Richtung hat wie die Verbindungslinie zwischen L1 und K1, entspricht der Winkel zwischen der Verbindungsgeraden F-K2 und dem Zentralstrahl dem Winkel $\beta 1$; $\beta 1$ ergibt sich zu

$$\beta 1 = atn(x1/a) \quad (3)$$

Für einen Punkt auf der Verbindungsgeraden F-K2 gelten die Gleichungen

$$x = z \cdot x1/a \quad (4)$$

und

$$y = z \cdot y1/a \quad (5)$$

Die Gleichungen (4) und (5) besagen, daß das Konkrement auf einer Geraden durch den Fokus F verschoben wird, wenn die Koordinaten x, y und z in der durch Gleichung (4) und (5) bestimmten Beziehung zueinander stehen. Man muß somit nur noch erkennen, wann sich das Konkrement F gerade im Fokus befindet und die Verschiebung dann beenden.

Dies wird mit Hilfe einer zweiten Röntgenaufnahme erreicht, vor der die Röntgenaufnahmeanordnung um einen Winkel b von z.B. 40° geschwenkt wird, der größer sein soll als die Hälfte des Öffnungswinkels, mit dem das Strahlenbündel die Eingangsebenen des Bildverstärkers vollständig erfaßt. Die zweite Röntgenaufnahme wird in dem digitalen Bildspeicher 10 gespeichert und auf dem Monitor 11 dargestellt, wobei u und v so vorgegeben werden, daß das Fadenkreuz 12 wieder seine Ursprungsposition (Bildmitte) einnimmt. Danach erfolgt der zweite Positioniervorgang, wobei die Tischplatte in allen drei Richtungen bewegt wird, bis das Fadenkreuz 12 die Abbildung des Konkrementes auf dem Monitor erreicht hat.

Es läßt sich zeigen, daß das Fadenkreuz auf dem Monitor mit der Abbildung des Konkrements zusammenfällt und gleichzeitig das Konkrement den Fokus erreicht, wenn folgende Bedingungen erfüllt werden:

1. Die Werte x und y werden entsprechend den Gleichungen (4) und (5) in Abhängigkeit von z geändert. Das bedeutet, daß sich auch x und y relativ zueinander in einem ganz bestimmten Verhältnis (x1/yl) ändern. Die sich für die x- und y-Richtung ergebenden Verschiebungen müssen zusätzlich zu den bereits während des ersten Positioniervorganges erfolgten Verschiebungen erfolgen. Infolgedessen müssen beim zweiten Positioniervorgang bei der Berechnung der Sollwerte für die x- und y-Positionen die Werte x1 bzw. y1 zu den nach Gleichung (4) bzw. (5) berechneten Werten addiert werden.

2. Zwischen v und u besteht der gleiche Zusammenhang wie zwischen y und x, d.h. es gilt die Gleichung:

$$v = u . y1/x1 \quad ( 6 )$$

3. Die Größen z und u, die ihrerseits die Tischplattenverschiebung (x, y, z) entsprechend den Gleichungen (4) und (5) und die Fadenkreuzverschiebung (u,v) nach Gleichung (6) bestimmen, hängen entsprechend der folgenden Gleichung zusammen:

$$z = u . A/(B + u . D) \quad ( 7 )$$

Dabei sind A, B und D Konstanten, die durch die Aufnahmeanordnung, d.h. durch die Werte a, c und d, den Schwenkwinkel b und den Winkel $\beta1$ bei der ersten Röntgenaufnahme bestimmt sind nach den Gleichungen

$$A = a . c . \cos(\beta1) \quad ( 8 )$$

$$B = d . \sin(b + \beta1) \quad ( 9 )$$

$$D = c . \cos(b + \beta1) \quad (10)$$

Der Schwenkwinkel b ist entweder von vornherein bekannt, wenn die Röntgenaufnahmeanordnung 5,6 jeweils in eine genau definierte Winkelstellung geschwenkt werden kann, oder er wird mittels eines geeigneten, mit dem Tragarm 8 gekoppelten Winkelgebers gemessen. Der Winkel $\beta1$ ergibt sich aus dem nach der ersten Aufnahme gemessenen und gespeicherten Wert x1 entsprechend Gleichung (3).

Gemäß den obengenannten Bedingungen sind bei dem zweiten Positioniervorgang u und v nicht mehr unabhängig voneinander wählbar. Infolgedessen wird bei dem zweiten Positioniervorgang v nicht unmittelbar über die Bedienungseinheit 14 vorgegeben, sondern von der Steuereinheit 13 aus dem Wert u entsprechend Gleichung (6) errechnet. Diese unterschiedliche Erzeugung von v bei den beiden Positioniervorgängen ist durch eine Umschalteinheit 15 schematisch angedeutet, die den einen Steuereingang des Monitors beim ersten Positioniervorgang mit dem Ausgang der Bedienungseinheit 14 und beim zweiten Positioniervorgang mit einem Ausgang der Steuereinheit 13 verbindet. Die Steuereinheit 13 berechnet aus dem Wert u weiterhin den Wert z nach Gleichung (7) bzw. die Werte x und y.

Die Berechnung kann schrittweise während des zweiten Positioniervorganges erfolgen, so daß in jeder Stellung die zusammengehörenden Werte von u, v, z, x und y für den nächsten Verschiebungsschritt von Fadenkreuz und Tischplatte berechnet werden. Dies setzt voraus, daß die Steuereinheit diese Berechnungen entsprechend schnell durchführt. Grundsätzlich ist es aber auch möglich, schon nach dem ersten Positioniervorgang für sämtliche Schritte die zusammengehörenden Wertepaare berechnen und zu speichern und beim zweiten Positioniervorgang die gespeicherten Wertepaare für die betreffende Position aufzurufen. Dies setzt allerdings eine entsprechende Speicherkapazität der Steuereinheit 13 voraus.

Es ist auch möglich, anstatt aus u den Wert z umgekehrt aus z den Wert u zu berechnen. Gemäß Gleichung (7) ist dies zwar nur durch ein numerisches Iterationsverfahren möglich, doch bietet die Ausführung eines solchen Verfahrens mit einer geeignet ausgebildeten Steuereinheit (Mikroprozessor) keine Schwierigkeiten. In diesem Fall gibt der Benutzer an einer entsprechend ausgebildeten Bedienungseinheit die Tischplattenverschiebung in z-Richtung vor und alle anderen Werte (x, y, u und v werden daraus abgeleitet), so daß der Benutzer auf diese Weise unmittelbar die Verschiebungsgeschwindigkeit bestimmen kann.

Bei dem Beispiel, dessen Geometrie in Fig. 3 dargestellt ist, wurde angenommen, daß das Konkrement sich unterhalb der durch den Fokus F definierten Ebene befindet. In einem solchen Fall wird das Konkrement bei der einen Röntgenaufnahme links und bei der anderen Röntgenaufnahme rechts vom Fadenkreuz 12 auf dem Monitor abgebildet. Infolgedessen kehrt sich bei den Positioniervorgängen das Vorzeichen u um. Wenn hingegen das Konkrement oberhalb von F, d.h. zwischen den durch den Bildverstärker B und den Fokus F bestimmten parallelen Ebenen läge, dann würde das Konkrement auch nach der zweiten Röntgenaufnahme auf der gleichen Seite des Fadenkreuzes abgebildet. In diesem Fall müßte u bei beiden Positioniervorgängen in der gleichen Richtung variiert werden. Andererseits müßte z im letztgenannten Fall in der entgegengesetzten Richtung verändert werden, wie bei einem unterhalb von F liegenden Konkrement. Das Vorzeichen der Verschiebung der Tischplatte in z-Richtung kann daher aus der Änderung von u beim ersten Positioniervorgang (bzw. - was auf dasselbe hinausläuft - aus dem Vorzeichen von x1) und dem Vorzeichen von u beim zweiten Positioniervorgang eindeutig abgeleitet werden.

Um den Patienten beim Schwenken des Bildverstärkers in die Position für die zweite Röntgenaufnahme nicht zu gefährden, kann durch Verschieben des Bildverstärkers 5 in Längsrichtung des Tragarms 8 der Abstand zwischen dem Bildverstärker und dem Fokus F vergrößert werden. In einem solchen Fall müssen die Konstanten A, B und D in Gleichung (7) der veränderten Geometrie angepaßt werden.

Die Steuereinheit 13 arbeitet digital; die gegebenenfalls erforderlichen D/A- oder A/D-Wandler, über die diese Einheit mit den anderen Einheiten zusammenarbeitet, sind in Fig. 2 nicht dargestellt. Vorzugsweise wird die Steuereinheit mit Hilfe eines Mikroprozessors realisiert, der nach einem geeigneten Programm die Positioniervorgänge steuert. In diesem Fall kann die in Fig. 2 durch eine Umschalteinrichtung 15 konkretisierte Änderung der Erzeugung des Signals v bei dem zweiten Positioniervorgang softwaremäßig realisiert werden. Der Mikroprozessor kann auch noch zusätzlich die Funktion der Regler Rxy und Rz übernehmen.

Das vorstehend erläuterte Verfahren wurde so durchgeführt, daß das Röntgenaufnahmesystem nach der ersten Röntgenaufnahme um einen vorgegebenen Winkel geschwenkt wurde, so daß sich bei den beiden Röntgenaufnahmen unterschiedliche Perspektiven ergeben. Wenn man bedenkt, daß das Konkrement auch infolge der Verschiebung vor der zweiten Röntgenaufnahme bei den beiden Röntgenaufnahmen aus unterschiedlicher Perspektive abgebildet wird, erkennt man, daß das Schwenken des Röntgenaufnahmesystems an sich nicht unbedingt erforderlich ist, um unterschiedliche Perspektiven zu erhalten.

Die zweite Röntgenaufnahme kann daher auch mit derselben Richtung des Zentralstrahls durchgeführt werden, d.h., das Röntgenaufnahmesystem muß nicht schwenkbar sein. Um unterschiedliche Perspektiven auch dann zu erzielen, wenn bei der ersten Aufnahme der Zentralstrahl zufällig schon durch das Konkrement verläuft, muß nach dem ersten Positioniervorgang und vor der zweiten Röntgenaufnahme die Tischplatte zusätzlich um eine definierte Strecke relativ zum Zentralstrahl - oder umgekehrt - verschoben werden. Diese zusätzliche Verschiebung muß dann beim zweiten Positioniervorgang (nach der zweiten Aufnahme) wieder rückgängig gemacht werden. Auch dafür gelten dann die einfachen Beziehungen nach den Gleichungen (1) und (2). Da die Unterschiede in der Perspektive aber geringer sind als bei einer Schwenkung des Röntgenaufnahmesystems, ist die erreichbare Positioniergenauigkeit dieser Abwandlung geringer.

**Patentansprüche**

1. Verfahren zum Positionieren eines auf der Tischplatte (3) eines Lagerungstisches befindlichen Patienten, wobei eine erste Röntgenaufnahme aus einer ersten Perspektive angefertigt und elektronisch gespeichert wird, wonach die Tischplatte in einem ersten Positioniervorgang in einer ersten (x) und in einer dazu senkrechten zweiten Richtung (y) verfahren wird und die Röntgenaufnahme und eine darin eingeblendete elektronische Marke relativ zueinander entsprechend dem Fahrweg der Tischplatte (3) verschoben werden, dadurch gekennzeichnet, daß eine zweite Aufnahme aus einer zweiten Perspektive angefertigt und elektronisch gespeichert wird und daß die Tischplatte danach in einem zweiten Positioniervorgang in einer zu den beiden ersten Richtungen senkrechten dritten Richtung (z)verfahren wird, wobei mit der Verschiebung in der dritten Richtung automatisch eine Verschiebung in der ersten und der zweiten Richtung gekoppelt ist, derart, daß der Fahrweg (x bzw. y) in der ersten und der zweiten Richtung dem Produkt aus dem Fahrweg (z) in der dritten Richtung und dem Fahrweg in der ersten bzw. zweiten Richtung (x1 bzw y1) bei dem ersten Positioniervorgang proportional ist, und daß der Zusammenhang zwischen der Relativverschiebung (u) der Marke und der Tischplattenverschiebung (z) aus den Fahrwegen nach der ersten Aufnahme berechnet wird und diese Verschiebungen entsprechend der Berechnung erfolgen.

2. Verfahren nach Anspruch 1, dadurch gekennzeichnet,daß die erste und die zweite Aufnahme mit in unterschiedlichen Richtungen verlaufendem Zentralstrahl angefertigt werden.

3. Verfahren nach Anspruch 1, dadurch gekennzeichnet, daß die beiden Röntgenaufnahmen mit der gleichen Richtung des Zentralstrahls angefertigt werden und daß nach dem ersten Positioniervorgang und vor der zweiten Röntgenaufnahme die Tischplatte um eine definierte Strecke in der ersten bzw. der zweiten Richtung verschoben wird.

4. Gerät zum Durchführen des Verfahrens nach Anspruch 1 mit einem Röntgenaufnahmesystem (5, 6) zum Anfertigen von Röntgenaufnahmen des Patienten mit unterschiedlicher Perspektive und zur elektronischen Speicherung der Röntgenaufnahme, mit einem Patientenlagerungstisch, dessen Tischplatte (3) durch Antriebsmotoren (Mx, My, Mz) in drei zueinander senkrechten Richtungen verfahrbar ist, gekennzeichnet durch eine Einrichtung (11) zur Verschiebung der Röntgenaufnahme und einer darin elektronisch eingeblendeten Marke (13) relativ zueinander entsprechend dem Fahrweg der Tischplatte in der jeweiligen Richtung und durch eine Steuereinheit, die die Fahrwege (x1, y1) der Tischplatte (3) nach einer ersten Aufnahme speichert und die nach einer zweiten Aufnahme die Verschiebung in einer zu den beiden Richtungen senkrechten dritten Richtung automatisch mit einer Verschiebung in der ersten und in der zweiten Richtung koppelt, derart, daß der Fahrweg (x bzw. y) in der ersten und der zweiten Richtung dem Produkt aus dem Fahrweg (z) in der dritten Richtung und dem Fahrweg in der ersten bzw. zweiten Richtung (x1 bzw. y1) proportional ist und die den Zusammenhang zwischen der Reativverschiebung (u) der Marke und der Tischplattenverschiebung (z) aus den Fahrwegen nach der ersten Aufnahme berechnet und die Verschiebung der Marke entsprechend steuert.

5. Gerät nach Anspruch 4, zur Durchführung des Verfahrens nach Anspruch 2, dadurch gekennzeichnet, daß das Röntgenaufnahmesystem einen Röntgenbildwandler (5) und einen darauf ausgerichteten Röntgenstrahler (6) umfaßt, und um eine Achse schwenkbar ist, die durch einen Punkt (F) verläuft, in dem eine bestimmte Region des Patienten zu positionieren ist.

6. Gerät nach einem der vorhergehenden Ansprüche 4 oder 5 , dadurch gekennzeichnet, daß es einen Stoßwellenerzeuger (1) zum Zertrümmern von Konkrementen im menschlichen Körper aufweist.

**Claims**

1. A method of positioning a patient lying on the top (3) of a patient table, where a first X-ray image is formed from a first perspective and is electronically stored, after which the table top is displaced in a first direction (x) and a second direction (y) which is perpendicular thereto during a first positioning operation, the X-ray image and an electronic marker superposed thereon being shifted relative to one another in accordance with the path of travel of the table top, characterized in that from a second perspective a second exposure is formed and electronically stored, the table top subsequently being displaced in a third direction (z), perpendicular to the first two directions, during a second positioning operation, the displacement in the third direction being automatically linked to a displacement in the first and the second direction so that the path of trave (x, y) in the first and the second direction is proportional to the product of the path of travel (z) in the third direction and the path of travel in the first and the second direction (x1, y1, respectively) during the first positioning operation, the relationship between the relative displacement (u) of the marker and the table top displacement (z) being calculated from the paths of travel after the first exposure, said displacements taking place in conformity with the calculation.

2. A method as claimed in Claim 1, characterized in that the first and the second image are formed with a central ray extending in different directions.

3. A method as claimed in Claim 1, characterized in that the two X-ray images are formed with the same direction of the central ray, the table top being displaced over a defined distance in the first or the second direction after the first positioning operation and before the second X-ray exposure.

4. An apparatus for performing the method claimed in Claim 1, comprising an X-ray imaging system (5, 6) for forming X-ray images of the patient from different perspectives and for electronically storing the X-ray image, a patient table whose top (3) is displaceable in three mutually perpendicular directions by drive motors (Mx, My, Mz) characterized in that it comprises a device (11) for shifting the X-ray image and a marker (13) electronically superposed thereon relative to one another in accordance with the path of travel of the table top in the relevant direction, and a control unit which stores the paths of travel (x1, y1) of the table top (3) after a first exposure, which, after a second exposure, couples the displacement in a third direction, perpendicular to the two directions, automatically to a displacement in the first and in the second direction so that the path of travel (x, y) in the first and the second direction is proportional to the product of the path of travel (z) in the third direction and the path of travel in the first and the second direction (x1, y1), respectively and which calculates the relationship between the relative displacement (u) of the marker and the table top displacement (z) from the paths of travel after the first exposure and controls the displacement of the marker accordingly.

5. An apparatus as claimed in Claim 4 for performing the method claimed in Claim 2, characterized in that the X-ray imaging system comprises an X-ray image converter (5) and an X-ray source (6) aligned with respect thereto and is pivotable about an axis which extends through a point (F) in which a given region of the patient is to be positioned.

6. An apparatus as claimed in any of the preceding Claims 4 or 5, characterized in that it comprises a shockwave generator (1) for crushing concrements in human bodies.

## Revendications

1. Procédé de positionnement d'un patient couché sur le plateau (3) d'une table de support, suivant lequel une première radiographie est prise selon une première perspective et est stockée électroniquement après quoi le plateau de la table est déplacé au cours d'une première opération de positionnement, dans une première direction (x) et dans une seconde direction (y) perpendiculaire à la première et la radiographie ainsi qu'un repère électronique superposé sont déplacés l'un par rapport à l'autre d'une manière correspondant au trajet de déplacement du plateau (3) de la table, caractérisé en ce qu'une seconde radiographie est prise selon une seconde perspective et est stockée électroniquement et que le plateau de la table est ensuite déplacé, au cours d'une seconde opération de positionnement, dans une troisième direction (z) perpendiculaire aux deux premières, le déplacement dans la troisième direction étant couplé automatiquement à un déplacement dans les première et deuxième directions, de telle sorte que le trajet de déplacement (x ou y) dans la première et la deuxième direction soit proportionnel au produit du trajet de déplacement (z) dans la troisième direction et du trajet de déplacement dans la première ou la deuxième direction (x1 ou y1) lors de la première opération de positionnement, et que la relation entre le déplacement relatif (u) du repère et le déplacement (z) du plateau de la table est calculée à partir des trajets de déplacement après la première radiographie et ces déplacements se déroulent conformément au calcul.

2. Procédé suivant la revendication 1, caractérisé en ce que la première et la seconde radiographie sont prises avec un rayon central s'étendant dans des directions différentes.

3. Procédé suivant la revendication 1, caractérisé en ce que les deux radiographies sont prises avec la même direction pour le rayon central et après la première opération de positionnement, mais avant la seconde radiographie, le plateau de la table est déplacé d'une distance définie dans la première ou dans la deuxième direction.

4. Appareil pour l'exécution du procédé suivant la revendication 1, comportant un système radiographique (5, 6) pour prendre des radiographies du patient avec des perspectives différentes et pour stocker électroniquement ces radiographies, et une table de support pour le patient dont le plateau (3) peut être déplacé dans trois directions perpendiculaires l'une à l'autre par des moteurs d'entraînement (Mx, My, Mz), caractérisé par un dispositif (11) pour déplacer la radiographie et un repère (13) superposé électroniquement dans celle-ci l'un par rapport à l'autre, conformément au trajet de déplacement du plateau de la table dans la direction en question et à l'intervention d'une unité de commande qui stocke les trajets de déplacement (x1, y1) du plateau (3) de la table après une première radiographie et qui, après une seconde radiographie, couple le déplacement dans une troisième direction perpendiculaire aux deux premières automatiquement à un déplacement dans la première et dans la deuxième direction, de telle sorte que le trajet de déplacement (x ou y) dans la première et la deuxième direction soit proportionnel au produit du trajet de déplacement (z) dans la troisième direction et du trajet de déplacement dans la première ou la deuxième direction (x1 ou y1), et qui calcule, la relation entre le déplacement relatif (u) du repère et le déplacement (z) du plateau de la table à partir des trajets de déplacement, après la première radiographie, et commande de manière adéquate le déplacement du repère.

5. Appareil suivant la revendication 4, pour exécuter le procédé suivant la revendication 2, caractérisé en ce que le système radiographique comprend un convertisseur d'image de rayons X (5) et un générateur de rayons X (6) orienté vers ce convertisseur et peut pivoter autour d'un axe qui passe par un point (F) dans lequel une région déterminée du corps du patient doit être positionnée.

6. Appareil suivant l'une ou l'autre des revendications 4 ou 5, caractérisé en ce qu'il comporte un générateur d'ondes de choc (1) pour pulvériser des concrétions dans le corps humain.

Fig.1

Fig.2

Fig.3